# FASCICULE DE BREVET EUROPEEN

(11) **EP 3 024 923 B1**
(45) Date de publication et mention de la délivrance du brevet: **05.07.2017**
(21) Numéro de dépôt: 14750775.0
(22) Date de dépôt: 24.07.2014
(51) Int. Cl.: C12N 1/00, C12N 1/12, C12N 1/02, C12N 1/06

(54) **PROCEDE D'OPTIMISATION DU RENDEMENT DE PRODUCTION, DE LA QUALITE ORGANOLEPTIQUE ET DE LA STABILITE DANS LE TEMPS D'UNE BIOMASSE DE MICROALGUES RICHES EN PROTEINES**
VERFAHREN ZUR LANGFRISTIGEN OPTIMIERUNG DER HERSTELLUNGSEFFIZIENZ, ORGANOLEPTISCHEN QUALITÄT UND STABILITÄT EINER PROTEINREICHEN MIKROALGENBIOMASSE
METHOD FOR OPTIMISING THE PRODUCTION EFFICIENCY, ORGANOLEPTIC QUALITY AND STABILITY OVER TIME OF A PROTEIN-RICH MICROALGAE BIOMASS

(30) Priorité: 25.07.2013 FR 1357326; 25.03.2014 FR 1452486
(43) Date de publication de la demande: 01.06.2016
(73) Titulaire: Roquette Frères, 62136 Lestrem (FR)
(72) Inventeur: DRUON, Amandine, 59000 Lille (FR); PATINIER, Samuel, 59890 Quesnoy-sur-Deûle (FR); TOURSEL, Béatrice, 62920 Gonnehem (FR)
(74) Mandataire: Cabinet Becker et Associés
(86) Numéro de dépôt international: PCT/FR2014/051918
(87) Numéro de publication internationale: WO 2015/011418

(56) Documents cités:
- US-A1- 2010 303 990
- DATABASE WPI Week 200942 Thomson Scientific, London, GB; AN 2009-J46993 XP002730563, & CN 101 429 467 A (QINGDAO BIOLOGICAL ENERGY SOURCE&PROCE) 13 mai 2009 (2009-05-13)

## Description

La présente invention est relative à un procédé d'optimisation du rendement de production, de la qualité organoleptique et de la stabilité dans le temps d'une biomasse de microalgues riches en protéines, microalgues du genre *Chlorella,* plus particulièrement *Chlorella vulgaris, Chlorella sorokiniana,* ou *Chlorella protothecoides.*

### Présentation de l'état de l'art

Il est bien connu de l'homme du métier que les chlorelles sont une source potentielle de nourriture, car elles sont riches en protéines et autres nutriments essentiels.

Elles renferment notamment 45% de protéines, 20% de matières grasses, 20% de glucides, 5% de fibres et 10% de minéraux et de vitamines.

L'utilisation des microalgues (et principalement leurs protéines) comme aliments est de plus en plus considérée pour trouver des sources alternatives à la demande croissante en protéines animales au niveau mondial (comme rapporté par la FAO).

L'Union européenne, depuis des années, souffre par ailleurs d'un déficit structurel en protéines végétales qui s'élève, ces dernières années, à plus de 20 millions de tonnes d'équivalent soja, aujourd'hui importés d'Amérique du Sud.

La production de masse de certaines microalgues riches en protéines est alors envisagée comme une possibilité de combler ce « déficit en protéines ».

Des analyses exhaustives et des études nutritionnelles ont montré que ces protéines d'algues sont équivalentes aux protéines végétales conventionnelles, voire de meilleure qualité.

Toutefois, en raison des coûts élevés de production ainsi que des difficultés techniques pour intégrer le matériel issu de microalgues dans des préparations alimentaires organoleptiquement acceptables, la diffusion large des protéines de microalgues est encore à ses balbutiements.

Des biomasses de microalgues de différentes espèces présentant un pourcentage élevé en protéines ont été rapportées (voir le tableau 1 de Becker, Biotechnology Advances (2007)25:207-210).

Un certain nombre de demandes de brevet dans l'état de l'art, telle la demande de brevet WO 2010/045368, enseignent quant à elles qu'il est possible d'ajuster les conditions de culture de manière à augmenter encore la teneur en protéines de la biomasse de microalgues.

De manière préférentielle, pour les microalgues qui en ont la capacité, la culture est réalisée en hétérotrophie, en l'absence de lumière et en présence d'une source de carbone assimilable.

Ces voies de croissance en hétérotrophie permettent à la fois de réaliser des productions en masse de microalgues, et d'en améliorer la qualité organoleptique en inhibant la synthèse par la microalgue de chlorophylle, à l'origine du gout prononcé de thé vert des préparations alimentaires en contenant.

Pour enrichir le contenu en protéines, la microalgue est cultivée dans un milieu enrichi en azote en présence d'une source carbonée abondante, telle le glucose. L'azote est alors indifféremment apporté par des sources organiques ou minérales.

La biomasse de microalgues ainsi produite comprend typiquement au moins 40%, voire jusqu'à 50 - 60 % de protéines en poids sec de cellules.

Cependant rien n'est acquis, au sens où, outre les travaux déployés pour les procédés amont (terme anglais de « *Upstream Process* ») de production de biomasses riches en protéines - notamment la recherche de conditions de fermentation adaptées - d'autres difficultés naissent du traitement aval de ladite biomasse (terme anglais de « *Downstream Process* ») pour l'incorporer dans les préparations alimentaires d'intérêt.

Classiquement, le traitement aval comprend plusieurs étapes :
- collecte des microalgues séparées de leur milieu de croissance,
- pasteurisation et lavage,
- éventuellement cassage ou rupture des cellules afin d'en libérer les molécules d'intérêt,
- séchage.

La première étape de collecte des cellules est réalisée par la mise en oeuvre d'une ou plusieurs étapes de séparation solide/liquide.

La biomasse est habituellement collectée par sédimentation, centrifugation ou filtration, et quelque fois une étape de floculation supplémentaire est nécessaire.

Suite à cette première étape, qui peut permettre de la concentrer de 50 à 200 fois, la suspension de microalgues doit être traitée rapidement à défaut de quoi elle se dégrade rapidement.

Une premier opération consiste à pasteuriser ladite suspension, c'est-à-dire la chauffer de manière à limiter ou inhiber la charge microbienne (croissance de bactéries contaminantes), mais aussi de manière à inactiver certaines enzymes susceptibles de générer des goûts ou odeurs indésirables (« Off-Flavors »).

Cette opération est conduite classiquement à haute température pendant un temps court (procédé dit « High Temperature Short Time » ou HTST ou Ultra Haute Température ou UHT)

Une seconde opération est le lavage, préconisée sur cellules intactes (avec des volumes d'eau distillée ou désionisée) de manière à éliminer les impuretés solubles.

Dans le cas où une étape de cassage ou rupture cellulaire est envisagée, plusieurs voies sont possibles : mécaniques (homogénéisateurs, broyage à billes ou ultrasons), ou non mécaniques (voie alcaline, cycles de congélation/décongélation, solvants organiques ou chocs osmotiques).

Le choix de la méthode est fonction de la nature de la paroi cellulaire de la microalgue à rompre, et de la nature du produit à isoler.

La dernière étape du traitement aval consiste en la déshydratation de ladite suspension (cellules intactes ou lysées). Plusieurs méthodes ont été employées pour sécher les microalgues du genre *Chlorella, Scenedesmus* et *Spirulina.* Les plus classiques sont l'atomisation, le séchage sur tambour sécheur, la lyophilisation. L'atomisation est la méthode la plus souvent utilisée à l'échelle industrielle.

Cependant, la sensibilité de certaine biomasse à l'oxydation impose l'addition d'antioxydants.

Malgré cette diversité de méthodes, et de combinaison de méthodes adaptées aux microalgues, il demeure six principales difficultés (énumérées a) à f) ci-dessous) non encore résolues de manière satisfaisante par l'homme du métier, en particulier :
a) la perte de rendement en protéines au cours du traitement aval *(Downstream Process),*
b) la perte de la teneur en protéines déplorée après l'étape de désactivation thermique de la biomasse (pouvant atteindre jusqu'à 25 % de perte),
c) la genèse de goûts ou odeurs indésirables (Off-Notes) non maitrisées, malgré les étapes de lavage préconisées,
d) l'absence de stabilité dans le temps des lots produits non encore expliquée, et de reproductibilité dans la stabilité puisque certains lots sont stables, et d'autres pas,
e) les risques de contaminations microbiennes du produit fini, et
f) la chute du rendement énergétique global du procédé de purification basé sur la mauvaise gestion du facteur de concentration de la biomasse.

### Résumé de l'invention

Pour remédier à ces difficultés, la société Demanderesse a choisi de conduire des travaux sur la mise en oeuvre d'étapes de traitement aval adaptées et dont l'efficacité peut être mesurée :
a) par le calcul du rendement et de la teneur en protéines de la biomasse produite et/ou la teneur en biomasse sèche, mais également
b) par des méthodes d'analyse sensorielle, et/ou
c) de mesure de la stabilité dans le temps des lots produits, grâce à une méthode de vieillissement accélérée tout à fait particulière, développée par la société Demanderesse.

La présente invention est relative à un procédé d'optimisation du traitement en aval d'une biomasse de microalgues du genre *Chlorella* riche en protéines préalablement préparée par fermentation en conditions hétérotrophiques et en absence de lumière, comprenant :
1) fournir une biomasse comprenant plus de 50 % de protéines en poids sec de biomasse ;
**puis, effectuer à** une température inférieure à 8°C, de préférence inférieure à 4°C :
2) la récolte de la biomasse en fin de fermentation,
3) le lavage et la concentration de la biomasse
4) facultativement, la lyse de la biomasse,
**puis, sans contrainte de** température inférieure à 8°C :
5) facultativement, concentrer la suspension de biomasse,
6) appliquer un traitement thermique,
7) sécher la biomasse ainsi obtenue pour obtenir le produit,
une étape d'ajustement du pH à 7 étant appliquée avant ou après l'étape 6) de traitement thermique.

De préférence, la biomasse comprend plus de 50 % en poids sec de protéines, de préférence plus de 55 %, plus préférentiellement encore plus de 60, 65 ou 70%.

Lorsque les étapes sont effectuées à basse température, la température est maintenue inférieure à 8°C, de préférence inférieure à 4°C. De préférénce, cette basse température est appliquée tout au long des étapes 2) à 4) du procédé conforme à l'invention.

De préférence, le traitement thermique est un traitement thermique Haute Température / Temps Court (HTST) pendant 30 secondes à 5 minutes à une température inférieure à 100 °C.

Alternativement, le traitement thermique est un traitement thermique Ultra Haute Température (UHT) à une température comprise entre 100 °C et 150 °C pendant 5 à 30 secondes.

De préférence, le lavage de la biomasse est réalisé avec au maximum 6 volumes d'eau pour 1 volume de biomasse, de préférence avec au maximum 3 volumes d'eau.

De préférence, la neutralisation de la suspension de biomasse à pH 7 est réalisée par ajout de KOH ou NaOH, de préférence par ajout de KOH.

De préférence, la lyse des cellules de la biomasse est réalisée par broyage, de préférence par broyage à billes.

De préférence, la concentration de la biomasse est réalisée par centrifugation ou évaporation.

Facultativement, les effets des étapes du traitement de la biomasse de microalgues sur la qualité du produit peuvent être déterminés par un ou plusieurs des paramètres suivants :
- mesure du poids sec de cellules dans la biomasse ;
- mesure de la teneur en sucres;
- détermination de la quantité de protéines
- analyse des composés organiques volatils;
- mesure d'activités enzymatiques, en particulier activité lipoxygénase ;
- mesure de la coloration ou de la teneur en pigments :
- mesure de la teneur en métaux, en particulier fer, cuivre ou nickel ;
- détermination du degré d'oxydation.

En particulier, les effets de plusieurs traitements de biomasse sont comparés pour la qualité du produit et le(s) traitement(s) donnant les meilleurs résultats sont sélectionnés.

Dans un mode de réalisation particulier, les microalgues du genre Chlorella sont choisies dans le groupe constitué de *Chlorella vulgaris, Chlorella sorokiniana* et *Chlorella protothecoides,* et sont plus particulièrement *Chlorella protothecoides.*

Le but de ce procédé est en particulier la mise au point d'un procédé optimisé de production de biomasse de microalgues riches en protéines à haut rendement, sans goûts ou odeurs indésirables et stable dans le temps.

### Description détaillée de l'invention

La présente invention est relative à un procédé optimisé permettant de satisfaire toutes les exigences propres à la production de biomasse de microalgues riches en protéines, plus particulièrement en termes de rendement de production en protéines, de qualité organoleptique et de stabilité dans le temps de ladite biomasse.

La présente invention est ainsi relative à un procédé d'optimisation du traitement en aval d'une biomasse de microalgues du genre *Chlorella* riche en protéines préalablement préparée par fermentation en conditions hétérotrophiques et en absence de lumière , comprenant :
1) fournir une biomasse comprenant plus de 50 % de protéines en poids sec de biomasse ;
**puis, effectuer à** une température inférieure à 8°C, de préférence inférieure à 4°C :
2) la récolte de la biomasse en fin de fermentation,
3) le lavage et la concentration de la biomasse
4) facultativement, la lyse de la biomasse,
**puis, sans contrainte de** température inférieure à 8°C :
5) facultativement, concentrer la suspension de biomasse,
6) appliquer un traitement thermique,
7) sécher la biomasse ainsi obtenue pour obtenir le produit,
une étape d'ajustement du pH à 7 étant appliquée avant ou après l'étape 6) de traitement thermique.

Selon l'étape 1) du procédé conforme à l'invention, la biomasse comprend au moins 50 % en poids sec de protéines. De manière encore plus préférée, elle comprend au moins 55, 60, 65 ou 70 % en poids sec de protéines.

Les microalgues préférées de l'invention peuvent croître en conditions hétérotrophiques (sur sucres comme source carbonée et en l'absence de lumière). La société Demanderesse recommande de choisir des microalgues du genre *Chlorella* riches en protéines. Les microalgues utilisées peuvent être choisies, et de manière non-exhaustive, parmi les *Chlorella protothecoides, Chlorella kessleri, Chlorella minutissima, Chlorella sp., Chlorella sorokiniama, Chlorella luteoviridis, Chlorella vulgaris, Chlorella reisiglii, Chlorella ellipsoidea, Chlorella saccarophila, Parachlorella kessleri, Parachlorella beijerinkii, Prototheca stagnora et Prototheca moriformis.* De manière préférée, les microalgues utilisées selon l'invention appartiennent à l'espèce *Chlorella protothecoides.*

Dans un mode très particulier, la souche de *Chlorella sorokiniana* est la souche UTEX 1663 - *The Culture Collection of Algae at the University of Texas at Austin-* USA. Dans un mode très particulier, la souche de *Chlorella protothecoides* est la souche CCAP211/8D- *The Culture Collection of Algae and Protozoa, Scotland, UK*.Les microalgues sont cultivées en milieu liquide pour produire la biomasse en tant que telle. Selon l'invention, les microalgues sont cultivées dans un milieu contenant une source de carbone et une source d'azote en absence de lumière (conditions hétérotrophiques). Les milieux de croissance solides et liquides sont généralement disponibles dans la littérature, et les recommandations pour la préparation des milieux particuliers qui conviennent à une grande variété de souches de microorganismes peuvent être trouvées, par exemple, en ligne à www.utex.org/, un site maintenu par l'Université du Texas à Austin pour sa collection de culture d'algues (UTEX). La production de biomasse est réalisée en fermenteurs (ou bioréacteurs).

Les exemples spécifiques de bioréacteurs, les conditions de culture, et la croissance hétérotrophe et les méthodes de propagation peuvent être combinés de toute manière appropriée pour améliorer l'efficacité de la croissance microalgale et la teneur en protéines. Les méthodes de production d'une telle biomasse sont bien connues de l'homme du métier.

Les étapes 2 à 4 du procédé conforme à l'attention, sont réalisées à basse température, c'est-à-dire à une température maintenue inférieure à 8°C, de préférence inférieure à 4°C. Cette basse température permet de stopper/freiner le métabolisme cellulaire ainsi que le développement de contaminants microbiens.

Par ailleurs, comme l'a remarqué la société Demanderesse, un autre avantage de cette conduite à basse température est que le refroidissement ainsi que l'oxygénation limitée favorisent la limitation des phénomènes oxydatifs générateurs des « *off notes* » et source d'instabilité du produit fini.

Plus particulièrement :
- Dans l'étape 2) du procédé conforme à l'invention, on récolte la biomasse en fin de fermentation.
   De manière avantageuse, on récolte la biomasse dès épuisement de la source nutritive résiduelle (en particulier du glucose résiduel). Ces conditions permettent d'optimiser les rendements de production de la biomasse produite, et limiter la concentration en solubles résiduels à éliminer lors de l'étape de lavage.
- Dans l'étape 3) du procédé conforme à l'invention, on effectue le lavage et la concentration de la biomasse.
   La biomasse est lavée des solubles résiduelles de fin de fermentation (sels, sucres non métabolisés,...) par dilution à l'eau.
   La biomasse est lavée avec au maximum 6 volumes d'eau par volume de biomasse, de préférence avec au maximum 3 volumes d'eau par volume de biomasse, et dans un mode de réalisation très particulier avec environ un volume d'eau par volume de biomasse.
   Cette opération permet d'améliorer significativement la pureté cellulaire (diminuer la part de matière sèche de fin de fermentation issue d'une composante non cellulaire).
   Par ce biais, la charge de ces solubles, potentiellement source de dégradation des propriétés sensorielles de la biomasse, est diminuée. Cette opération est avantageusement menée dans des conditions de basse température.
   La biomasse est ensuite concentrée à 15 à 40 % de matière sèche, de préférence à 20-30 % de matière sèche.
   Elle peut être concentrée par centrifugation, par exemple en utilisant une centrifugeuse Alfa Laval FEUX 510.
- Dans l'étape 4, de manière facultative, on lyse la biomasse ainsi obtenue.
   Les parois cellulaires et les composants intracellulaires sont broyés ou réduits.
   Différentes techniques sont disponibles pour réaliser la lyse comme le broyage par microbilles et la technologie d'homogénéisation à haute pression.
   Le mode préféré est le broyage par microbilles, en particulier broyage par broyage à bille « Bead Mill ».
   Classiquement, un broyeur à billes NETZSCH Labstar est utilisé avec des billes de silicate de zirconium de 0,5mm de diamètre. Le taux de lyse peut être variable. Par exemple, un taux de lyse de 50, 60, 70, 80, 90 ou 95 % des cellules peut être envisagé.

Les trois dernières étapes du procédé conforme à l'invention sont réalisées sans contrainte de basse température.

Dans l'étape 5), de manière facultative, on concentre la suspension de biomasse ainsi obtenue.
La biomasse est concentrée par évaporation. Tout type d'évaporateur peut être mis en oeuvre, par exemple un évaporateur rotatif, à flux forcé, à flot tombant ou à film raclé.
La concentration par évaporation participe à l'amélioration du facteur de concentration avant séchage en optimisant les performances énergétiques du procédé. Cette concentration permet également une élimination (« stripping ») des volatils potentiellement néfastes aux propriétés sensorielles du produit fini.
- Dans l'étape 6), on applique un traitement thermique,
   Ce traitement thermique agit comme une sécurité, face aux risques microbiologiques sur le produit fini.
   Classiquement, il consiste un traitement HTST ou UHT. Par ailleurs, ce traitement thermique participe à l'amélioration des propriétés sensorielles du produit fini.
   Deux types distincts de traitements thermiques sont envisagés en particulier. Le premier type est un traitement thermique Haute Température / Temps Court (HTST) de la biomasse, par exemple pendant 30 secondes à 5 min à une température inférieure à 100°C.
   Le deuxième type est un traitement thermique UHT (Ultra Haute Température). De préférence, le traitement thermique UHT est mené à une température comprise entre 100 et 150°C pendant 5 à 30 secondes, de préférence à une température comprise entre 120 et 140°C pendant 5 à 15 secondes.
- Dans l'étape 7), on sèche la biomasse ainsi obtenue pour obtenir le produit.
   De manière préférentielle, on effectue le séchage par atomisation. L'atomisation est réalisée dans un atomiseur où une suspension liquide est pulvérisée sous la forme d'une dispersion de fines gouttelettes dans un courant d'air chauffé, le matériel entraîné étant rapidement séché et forme une poudre sèche.
   Il existe de multiples dispositifs de séchage de composés riches en lipides par atomisation dans l'état de l'art. On peut trouver aisément dans la littérature des illustrations des technologies et appareillages proposés : par exemple dans le Spray Drying Handbook, de K. MASTERS, notamment dans sa 5ème édition, publiée en 1991 et republiée en 1994 par Longman Scientific & Technical (disponible à la British Library ou à la Library of Congress sous ISBN 0-470-21743-X) ou dans le BETE^{®} Spray Dry Manual, 2005 (accessible à l'adresse internet www.bete.com).
   Par exemple, l'atomisation peut être réalisée sur une tour d'atomisation simple effet NIRO-Minor Mobil ou sur un Filtermat FMD125 avec un cyclone.

Une dernière étape clef consiste, avant ou après l'étape 6) de traitement thermique, à neutraliser la suspension de biomasse (non lysée ou lysée) à pH 7.

Cette neutralisation peut être faite par ajustement du pH à 7 par ajout de NaOH ou KOH, de préférence de KOH. Cette neutralisation à la potasse concentrée permet de lisser les fluctuations possibles de pH en aval et entre les lots de production, mais également d'améliorer les propriétés sensorielles.

Il peut être également choisi d'ajouter un ou plusieurs antioxydants (avant ou après l'étape de neutralisation du pH à une valeur de 7).

On peut avantageusement choisir de l'acide ascorbique et/ou un mélange de tocophérols, de préférence une combinaison d'acide ascorbique et de tocophérols.

Classiquement, les proportions utilisées sont de 150 ppm/sec d'acide ascorbique et 500 ppm/sec d'un mélange de tocophérols. (x ppm/sec signifiant x mg par kg de biomasse sèche).

Il est bien entendu que la nature de l'antioxydant dépend des propriétés de la matrice à stabiliser. Ils doivent améliorer la stabilité du produit fini face aux risques d'altération oxydative et ainsi améliorer sa conservation en maintenant une stabilité du profil physico-chimique et sensoriel.

Il peut être par ailleurs déterminé les effets des étapes du traitement de la biomasse de microalgues sur la qualité du produit par :
∘ la mesure de la perte de rendement, en particulier l'analyse de la perte de matière sèche cellulaire ainsi que la perte de teneur protéique provenant notamment de la « solubilisation » durant le traitement thermique et son élimination au lavage si cette dernière étape est placée en aval.
   Il est démontré que cette perte de matière sèche est majoritairement constituée d'une fraction protéique (ce qui permet de répondre aux difficultés a) et b) identifiées précédemment) ;
∘ la détermination la qualité sensorielle des lots produits, notamment par un panel sensoriel formé pour évaluer des propriétés sensorielles de différents lots (pour répondre à la difficulté c)) ;
∘ la mesure de la stabilité dans le temps, notamment par un test de vieillissement accéléré, consistant en une analyse sensorielle comparative de l'échantillon initial et de ce même échantillon placée 10 jours à 60°C à l'étuve en conditionnement hermétique (pour répondre à la difficulté d)) ;
   L'analyse sensorielle est effectuée conformément au test décrit. Cette analyse permet de mettre en évidence des descripteurs éventuels d'oxydation, permettant ainsi d'évaluer la stabilité de l'échantillon face à cette dégradation oxydative.
∘ de mesurer l'évolution de la charge microbienne au cours du temps sur les différentes étapes du procédé (pour répondre à la difficulté e)).
∘ l'analyse de l'évolution de la matière sèche (facteur de concentration) au cours de cet enchainement d'opération (pour répondre à la difficulté f)).

Trois caractéristiques essentielles à l'évaluation de la qualité ont été définies par la Société Demanderesse :
- la teneur en biomasse sèche et/ou de protéines dans le produit;
- la qualité organoleptique du produit ; et
- la stabilité dans le temps du produit.

Par ailleurs, d'autres paramètres peuvent également pris en compte pour évaluer la qualité du produit, notamment les suivants :
- mesure du poids sec de cellules dans la biomasse ;
- mesure de la teneur en sucres;
- détermination de la quantité de protéines
- analyse des composés organiques volatils;
- mesure d'activités enzymatiques, en particulier activité lipoxygénase ;
- mesure de la coloration ou de la teneur en pigments :
- mesure de la teneur en métaux, en particulier fer, cuivre ou nickel ;
- détermination du degré d'oxydation.

L'invention sera mieux comprise à l'aide des exemples qui suivent, lesquels se veulent illustratifs et non limitatifs.

### EXEMPLES

Plusieurs lots ont été produits par traitement en aval d'une biomasse de *Chlorella protothecoides* préparée par fermentation en conditions hétérotrophiques et en absence de lumière. La souche utilisée est la *Chlorella protothecoides* référencée *UTEX 250.*

Les différentes étapes ont été réalisés telles que définies ci-dessous.

Traitement HTST : traitement thermique Haute Température / Temps Court (HTST) de la biomasse, pendant 30 secondes à 5 min, à une température inférieure à 100°C, notamment pendant 1 minutes à 75°C.

Lavage : avec au maximum 6 volumes d'eau par volume de biomasse.

Ajout d'antioxydant : ajout de d'acide ascorbique et d'un mélange de tocophérols, de préférence avec les proportions de 150 ppm/sec d'acide ascorbique et 500 ppm/sec d'un mélange de tocophérols.

Atomisation : atomisation sur une tour d'atomisation simple effet NIRO-Minor Mobil ou sur un Filtermat FMD125 avec un cyclone.

Broyage : broyage par broyage à bille « Bead Mill ». Classiquement, un broyeur à billes NETZSCH Labstar est utilisé avec des billes de silicate de zirconium de 0,5mm de diamètre.

Concentration : concentration / évaporation par évaporateur rotatif (à l'échelle laboratoire) ou tout autre type d'évaporateur à plus grande échelle (flux forcé, flot tombant, film raclé...) de 20 à 30 % de matière sèche.

Traitement UHT : à une température comprise entre 100 et 150°C pendant 5 à 30 secondes, de préférence à une température comprise entre 120 et 140°C pendant 5 à 15 secondes.

La qualité des lots obtenus est étudiée de la manière suivante. Un ou plusieurs paramètres suivants ont été déterminés ou mesurés.

La qualité sensorielle : la qualité sensorielle des lots produits est évaluée par un panel sensoriel d'environ 18 personnes sur un ensemble de descripteurs sensoriels. Le panel expert évalue les propriétés olfactives des lots à 3% dans de l'eau à 55°C (échantillons présentés dans un pot en verre fermé) sur des échelles d'intensité ordinale (NF V 09-015 : 1985).

La stabilité dans le temps : elle est mesurée lors d'un test de vieillissement accéléré mis au point par la société Demanderesse consistant en une analyse sensorielle comparative de l'échantillon initial et de ce même échantillon placée 10 jours à 60°C à l'étuve en conditionnement hermétique. L'analyse sensorielle permet de mettre en évidence des descripteurs éventuels d'oxydation, permettant ainsi d'évaluer la stabilité de l'échantillon face à cette dégradation oxydative.

### La mesure de perte de rendement :

- Mesure du poids sec de cellules (DCW). Et/ou
- Mesure de la biomasse sèche. Et/ou
- La teneur en protéines.

Par ailleurs, des paramètres supplémentaires peuvent également être évalués.

La teneur en sucres : Détermination de la teneur en sucres (glucose, maltose, fructose, saccharose) par chromatographie liquide. Apres séparation par chromatographie d'échange d'ions, les différentes espèces sont détectées par ampéromètrie.

Composés organiques volatils : La teneur en composés organiques volatils est déterminée par SPME / GC.

Analyse du traitement thermique : par observation les changements de morphologie cellulaires induits en microscopie optique.

Mesure de la coloration : Mesure à l'aide d'un spectrocolorimètre des mesures de réflectance aux longueurs d'onde de 400 nm à 700 nm sous l'illuminant D65 ou l'illuminant C et avec l'observateur CIE 1931 (2°). Détermination des indices « L », « a », « b » où « L » correspond à la luminance, « a » correspond à l'échelle du vert au rouge et « b » à l'échelle du bleu au jaune.

La teneur en pigments : Après cassage des cellules, les pigments sont extraits à l'acétone 90%. L'extrait est ensuite analysé par spectrophotométrie. La quantification des pigments est réalisée par calculs à partir des absorbances relevées à différentes longueurs d'ondes.

Le dosage de métaux : Destruction des matières organiques par minéralisation à l'aide d'un mélange sulfonitrique et détermination, après dilution appropriée, par spectrométrie d'émission.

Détermination du degré d'oxydation : Apres dilution dans l'Isooctane, mesure de l'absorbance à 232 nm.

## Revendications

1. Procédé d'optimisation du traitement en aval d'une biomasse de microalgues du genre *Chlorella* riche en protéines préalablement préparée par fermentation en conditions hétérotrophiques et en absence de lumière , comprenant :
1) fournir une biomasse comprenant plus de 50 % de protéines en poids sec de biomasse ;
**puis, effectuer à une** température inférieure à 8°C, de préférence inférieure à 4°C :
2) la récolte de la biomasse en fin de fermentation,
3) le lavage et la concentration de la biomasse,
4) facultativement, la lyse de la biomasse,
**puis, sans contrainte de température** inférieure à 8°C :
5) facultativement, concentrer la suspension de biomasse,
6) appliquer un traitement thermique,
7) sécher la biomasse ainsi obtenue pour obtenir le produit,
une étape d'ajustement du pH à 7 étant appliquée avant ou après l'étape 6) de traitement thermique.

2. Procédé selon la revendication 1, **caractérisé en ce que** la biomasse comprend plus de 60 % de protéines en poids sec de biomasse, de préférence plus de 65 ou 70 %.

3. Procédé selon l'une ou l'autre des revendications 1 et 2, **caractérisé en ce que** le traitement thermique est un traitement thermique Haute Température / Temps Court (HTST) pendant 30 secondes à 5 minutes à une température inférieure à 100 °C.

4. Procédé selon l'une ou l'autre des revendications 1 et 2, **caractérisé en ce que** le traitement thermique est un traitement thermique Ultra Haute Température (UHT) à une température comprise entre 100 °C et 150 °C pendant 5 à 30 secondes.

5. Procédé selon l'une quelconque des revendications 1 à 4, **caractérisé en ce que** le lavage de la biomasse est réalisée avec au maximum 6 volumes d'eau pour 1 volume de biomasse, de préférence avec au maximum 3 volumes d'eau.

6. Procédé selon l'une quelconque des revendications 1 à 5, **caractérisé en ce que** la neutralisation de la suspension de biomasse à pH 7 est réalisée par ajout de KOH ou NaOH.

7. Procédé selon l'une quelconque des revendications 1 à 6, **caractérisé en ce que** la lyse des cellules de la biomasse est réalisée par broyage, de préférence par broyage à bille.

8. Procédé selon l'une quelconque des revendications 1 à 7, **caractérisé en ce que** la concentration de la biomasse est réalisée par centrifugation ou évaporation.

9. Procédé selon l'une quelconque des revendications 1 à 8, **caractérisé en ce que** les effets des étapes du traitement de la biomasse de microalgues sur la qualité du produit sont également déterminés par un ou plusieurs des paramètres suivants :
- mesure du poids sec de cellules dans la biomasse ;
- mesure de la teneur en sucres;
- détermination de la quantité de protéines
- analyse des composés organiques volatils;
- mesure d'activités enzymatiques, en particulier activité lipoxygénase ;
- mesure de la coloration ou de la teneur en pigments :
- mesure de la teneur en métaux, en particulier fer, cuivre ou nickel ;
- détermination du degré d'oxydation.

10. Procédé selon l'une quelconque des revendications 1 à 9, **caractérisé en ce que** les microalgues du genre Chlorella sont choisies dans le groupe constitué de *Chlorella vulgaris, Chlorella sorokiniana* et *Chlorella protothecoides,* et sont plus particulièrement *Chlorella protothecoides.*

## Patentansprüche

1. Verfahren zur Optimierung der nachgelagerten Aufarbeitung einer proteinreichen Biomasse von Mikroalgen der Gattung *Chlorella,* die zuvor mittels Fermentation unter heterotrophen Bedingungen und unter Lichtabschluss produziert wurde, umfassend:
1) Bereitstellung einer Biomasse, umfassend mehr als 50% Proteine bezogen auf das Trockengewicht der Biomasse;
dann, Durchführen bei einer Temperatur unter 8°C, vorzugsweise unter 4°C:
2) Ernte der Biomasse am Ende der Fermentation,
3) Waschen und Aufkonzentrierung der Biomasse,
4) gegebenenfalls Lyse der Biomasse,
dann, ohne Beschränkung der Temperatur auf unter 8°C:
5) gegebenenfalls Aufkonzentrieren der Biomassesuspension,
6) Durchführen einer thermischen Behandlung,
7) Trocknen der auf diese Weise erhaltenen Biomasse, um das Produkt zu erhalten,
wobei ein Schritt der Einstellung des pH auf 7 vor oder nach Schritt 6) der thermischen Behandlung durchgeführt wird.

2. Verfahren gemäß Anspruch 1, **dadurch gekennzeichnet, dass** die Biomasse mehr als 60 Trockengew.-% Proteine, vorzugsweise mehr als 65% oder 70%, umfasst.

3. Verfahren gemäß einem der Ansprüche 1 und 2, **dadurch gekennzeichnet, dass** die thermische Behandlung eine kurzzeitige Hochtemperatur-(HTST)-Behandlung von 30 Sekunden bis 5 Minuten bei einer Temperatur unter 100°C ist.

4. Verfahren gemäß einem der Ansprüche 1 und 2, **dadurch gekennzeichnet, dass** die thermische Behandlung eine Ultrahochtemperatur-(UHT)-Behandlung bei einer Temperatur zwischen 100°C und 150°C von 5 bis 30 Sekunden ist.

5. Verfahren gemäß einem der Ansprüche 1 bis 4, **dadurch gekennzeichnet, dass** das Waschen der Biomasse mit maximal 6 Volumenteilen Wasser pro 1 Volumenteil Biomasse, vorzugsweise mit maximal 3 Volumenteilen Wasser, erfolgt.

6. Verfahren gemäß einem der Ansprüche 1 bis 5, **dadurch gekennzeichnet, dass** die Neutralisation der Biomassesuspension auf pH 7 durch Zugabe von KOH oder NaOH erfolgt.

7. Verfahren gemäß einem der Ansprüche 1 bis 6, **dadurch gekennzeichnet, dass** die Lyse der Zellen der Biomasse durch Mahlen, vorzugsweise durch Mahlen in einer Kugelmühle, erfolgt.

8. Verfahren gemäß einem der Ansprüche 1 bis 7, **dadurch gekennzeichnet, dass** die Aufkonzentrierung der Biomasse durch Zentrifugation oder Verdampfung erfolgt.

9. Verfahren gemäß einem der Ansprüche 1 bis 8, **dadurch gekennzeichnet, dass** die Effekte der Schritte der Behandlung der Biomasse von Mikroalgen auf die Qualität des Produkts ebenfalls mit einem oder mehreren der folgenden Parameter bestimmt werden:
- Messung des Zelltrockengewichts der Biomasse;
- Messung des Zuckergehalts;
- Bestimmung der Proteinmenge;
- Analyse der flüchtigen organischen Verbindungen,
- Messung von Enzymaktivitäten, insbesondere Lipoxygenaseaktivität;
- Messung der Färbung oder des Pigmentgehalts;
- Messung des Metallgehalts, insbesondere Eisen, Kupfer oder Nickel;
- Bestimmung des Oxidationsgrads.

10. Verfahren gemäß einem der Ansprüche 1 bis 9, **dadurch gekennzeichnet, dass** die Mikroalgen der Gattung *Chlorella* aus der Gruppe ausgewählt sind, bestehend aus *Chlorella vulgaris, Chlorella sorokiniana* und *Chlorella prothothecoides,* und insbesondere *Chlorella prothothecoides* sind.

## Claims

1. A method for optimizing the downstream processing of a protein-rich biomass of microalgae of the *Chlorella* genus which has been prepared beforehand by fermentation in heterotrophic conditions and in the absence of light, comprising:
1) providing a biomass comprising more than 50% proteins by dry weight of biomass;
then, at a temperature lower than 8°C, preferably lower than 4°C:
2) recovering the biomass at the end of fermentation,
3) washing and concentrating the biomass,
4) optionally lyzing the biomass,
then, with no temperature constraints lower than 8°C:
5) optionally concentrating the biomass suspension,
6) applying a heat treatment,
7) drying the resulting biomass, to obtain the product,
a step of adjusting the pH to 7 being applied before or after step 6) of heat treatment.

2. The method as claimed in claim 1, **characterized in that** the biomass comprises more than 60% proteins by dry weight of biomass, preferably more than 65 or 70%.

3. The method as claimed in either of claims 1 and 2, **characterized in that** the heat treatment is a high temperature/short time (HTST) heat treatment for 30 seconds to 5 minutes at a temperature lower than 100°C.

4. The method as claimed in either of claims 1 and 2, **characterized in that** the heat treatment is an ultra-high temperature (UHT) heat treatment at a temperature of between 100°C and 150°C for 5 to 30 seconds.

5. The method as claimed in any one of claims 1 to 4, **characterized in that** the biomass is washed with at most 6 volumes of water per 1 volume of biomass, preferably with at most 3 volumes of water.

6. The method as claimed in any one of claims 1 to 5, **characterized in that** the biomass suspension is neutralized to pH 7 by adding KOH or NaOH.

7. The method as claimed in any one of claims 1 to 6, **characterized in that** the cells of the biomass are lyzed by milling, preferably bead milling.

8. The method as claimed in any one of claims 1 to 7, **characterized in that** the biomass is concentrated by centrifugation or evaporation.

9. The method as claimed in any one of claims 1 to 8, **characterized in that** the effects of the steps of processing the microalgal biomass on the quality of the product are also determined by one or more of the following parameters:
- measuring the dry cell weight in the biomass;
- measuring the sugar content;
- determining the amount of proteins;
- analyzing the volatile organic compounds;
- measuring enzyme activities, in particular lipoxygenase activity;
- measuring the coloration or the pigment content;
- measuring the content of metals, in particular iron, copper or nickel;
- determining the degree of oxidation.

10. The method as claimed in any one of claims 1 to 9, **characterized in that** the microalgae of the *Chlorella* genus are chosen from the group consisting of *Chlorella vulgaris, Chlorella sorokiniana* and *Chlorella protothecoides,* and are more particularly *Chlorella protothecoides.*
